# EUROPEAN PATENT SPECIFICATION

(11) **EP 1 303 298 B1**
(45) Date of publication and mention of the grant of the patent: **27.09.2006**
(21) Application number: 01901073.5
(22) Date of filing: 25.01.2001
(51) Int. Cl.: A61K 38/51, A61K 38/54, A61K 31/355, A61K 9/00

(54) **PHARMACEUTICAL COMPOSITION COMPRISING CARRIERS FOR PHARMACOLOGICALLY ACTIVE PRODUCTS WHEREIN THE CARRIERS ARE BASED ON VITAMIN-E, PAPAIN AND HYALURONIDASE**
PHARMAZEUTISCHE ZUSAMMENSETZUNG ENTHALTEND TRÄGER FÜR PHARMAKOLOGISCH AKTIVE PRODUKTE WOBEI DIE TRÄGER AUF VITAMIN E, PAPAIN UND HYALURONIDASE BASIEREN
COMPOSITION PHARMACEUTIQUE COMPRENANT DES TRANSPORTEURS DE PRODUITS ACTIVES PHARMACOLOGIQUES DONT LES TRANSPORTEURS SONT BASE A VITAMINE E, DE PAPAINE ET D'HYALURONIDASE

(30) Priority: 28.01.2000 BR 0004260
(43) Date of publication of application: 23.04.2003
(73) Proprietor: Topic Empreendimentos e Participaçoes S/C Ltda., CEP-06453-000 Barueri, SP (BR)
(72) Inventor: SANTANA, Cristiano Alberto Ribeiro, CEP-01250-000 Sao Paulo, SP (BR); DE NUCCI, Gilberto, CEP-13092-320 Sao Paulo, SP (BR); FALCI, Marcio, CEP-05641-900 Sao Paulo, SP (BR)
(74) Representative: Prato, Roberto
(86) International application number: PCT/BR2001/000011
(87) International publication number: WO 2001/054647

(56) References cited:
- WO-A1-01/60399
- WO-A1-94/22423
- US-A- 5 914 322
- DATABASE WPI Week 200031, Derwent Publications Ltd., London, GB; AN 2000-351001, XP002958658 & BR 9 801 985 A (RIBEIRO SANTANA C.A.) 08 February 2000

## Description

The present invention refers to the use of papain, vitamin-E and hyaluronidase for manufacturing of a new pharmaceutical composition, to be used in any pharmaceutical form, most notably gel, cream, aerosol and spray, liquid and liophilizated, of a carrier substance for products to aggregate vitamin-E, papain and HYALURONIDASE. The above mentioned pharmaceutical composition is of topical application, non-toxic and features a high penetration rate through the skin.

### BRIEF DESCRIPTION OF THE INVENTION

The skin permeability varies according to the region of the body, being the skin folds and the face those that present the highest absorption rate. A product applied over the skin will present a longer period of contact and percutanial absorption.

According to the classic book "Histologia dos epiteliums", by Walter A. Hadler and Sineli R. Silveira, Editora Campus, Campinas, 1993, it is considered that: "bearing in mind the general morphological characteristics and the specialized functions that they perform, the epitelium cells are predominantly classified into two categories, which correspond to two epitelium classes: coating epitelium cells and secreting epitelium cells. The cells of these two classes mix with each other to constitute, respectively, the coating epiteliums and the secreting epiteliums, each one of them performing specific functions that are inherent to them. Such division is also fundamented in the distribution of these two classes of epitelium in the organism, which altough wide is is distinctive for both. With the purpose of forming the coating epiteliums the epitelium cells associate side-by-side, so as to originate "membranes" or layers superimposed over the base membrane, which function is to coat surfaces. On the contrary, the secreting cells unite to form organized functional units, better suited for performing their specialized function, related to the secretion products synthesys; thus are constituted the secreting units. The coating epiteliums are defined as living membranes, usually featuring a discontinuity, that isolate the organism from the environment, separating the internal media from the external one. Furthermore, these epiteliums isolate from each other the various internal media compartments, among which are the intravascular compartment, the serum compartment and several others. Among the various functions performed by the coating epiteliums some are performed by specialized variants that are specifically adapted to perform one or more functions. Others are incorporated as general functions presented without distinction by every coating epitelium cell. The coating epitelium cell, in the same way as most of the living cells, passively absorbs water and electrolytes and eliminates them actively; this function is well developed in the epitelium cells. On that account it is very important to observe that generally it is understood as absorption the penetration of solutions through the cells plasmatic membrane. However two different specific forms of absorption must be distinguished from one another: the passive absorption, that occurs according to the osmotic laws, and the active absorption, that entails the effective participation of the epitelium cell and that does not follow such physic laws. On the other hand it must be considered that every single substance that penetrates the interior of a multicellular organism, or else is excreted or elliminated, must cross at least one coating epitelium, because every superior organism is penetrated internally and externally by epiteliums. It must also be observed that the coating epiteliums, altough continuously covering and protecting those surfaces it coats, are not impervious at all; that is why they do not behave as inert "membranes". On the contrary, they allow for the exchange of gases, water, several kinds of electrolytes and certain other solutes between the internal and the external media, or between the various internal compartments, which characterizes its permeability. The coating epitelium cells limit in a controlled and selective way the permeability of the respective epiteliums, with the purpose of protecting the organism and still participate of the control of its homeostasis. In order to perform such function the epiteliums are organized and arrange their cells in a special form, in order to build up coatings which cells abbut the base membrane and are united with each other by means of intracellular junstions; in turn the cells are coated by the plasmatic membrane, which features special characteristics, and by the glycocup, both able to express well defined functional properties. The functional characteristics expressed by the plasmatic membrane portion that coats the cells apical surface are different from those expressed by the portion situated in its basal or basolateral face; such differences, which occur mainly on the funsctional aspect, contribute for the remarkable degree of polarization expressed by the coating epitelium cells. The prime function performed by the coating epiteliums correspond essentialy to the protection rendered to the surface that they coat, characterizing their protective coating function. Such function features a special characteristic, being a coating that, besides offering mechanical, physical and chemical protection to the coated surface, is not inert. The coating epiteliums are pervious, which allows for the controlled and selective passage of several products through its wall. There are many evidences in favor of the idea that the coating epiteliums permeability constitutes a fundamental property, with significant functional expression, for it is essential for the performance of several functions featured by the epiteliums, even more so because it is selective and its permeability degree presents a wide variation. It is fairly well demonstrated that the permeability degree influences strongly the function performed by the coating epiteliums:
1) wide permeability;
2) reduced permeability and
3) absence of permeability.

When there is a wide permeability, the epiteliums allow intense metabolic exchenges through their walls, with poor control and selectivity of its permeability. In these circumstances the epitelium acts on the filtration and transfer of metabolytes, these functions requiring little qualitative control; the exercise of these functions is subordinated to the epitelium intrinsec structure, which is adapted to act, mainly passively, being low the level of selective permeability. The coating epiteliums whith a reduced degree of permeability, due to the characteristic that is so peculiar to them, present the property of partially controlling their own permeability, and above all their selectivity. As a consequence, these coating epiteliums present selective permeability, which allows them to interfere and qualitatively control their functional activity, as well as making them more able to actuate over the homeostasis control. The absence of epitelium permeability is correlated to the complex isolation of the coated surface and, on the other hand, to the better controlling of this epitelium function, because its cells, altough very poorly pervious, present selective permeability. In this case the coated surface has its boundaries limited by a "membrane" impervious or very poorly pervious and very effective, that performs an important protective function, for it is able to discriminate exactly what can cross the epitelium. The coating epiteliums permeability is such an expressive functional property that it has been used as an important classification criterium to rank them in three classes:
1) pervious epiteliums;
2) poorly pervious epiteliums and
3) impervious epiteliums.

Because of their selective permeability, even in the inferior animals the epiteliums have assumed.the function of coating the organism, constituting its external coating, with limiting and protective properties, not only morphological but also functional. Their cells, in principle very similar, behaved as a semi-pervious "membrane" poorly effective that acted passively, but which function allowed the separation, tough precarious and more morphological than functional, between the internal and the external media. It seem to be that the majority of the coating epiteliums acts as a barrier that prevents the free passive diffusion, because their permeability, which is selective, is conditioned to several factors among which stands out the electric potential present in their cell's plasmatic membrane. The continuity of the epitelium coating is established as much through the intimate abutment of adjacent cells as through the presence of intercellular union devices. The epitelium cells are enveloped by the glycocup, that also takes part of the coating function performed by the epitelium, in adition to aid the union between adjacent cells, because the intracellular adhesive is formed also by glycocup. Several experimental investigations confirm that the coating epiteliums selective permeability is associated to other specific functions expressed by their cells, namely: absorption, excretion and secretion. These functions, beyond their permeability which constitutes their prime function, are responsible by the general functioning of the epitelium cell. The general functions performed by the coating epiteliums are basicaly the following:
1) surfaces protective coating function;
2) isolation and functional individualization of the internal media and of its distinct compartments, due to their cells selective permeability;
3) controlling the homeostasis of the internal medium and its compsrtments due to their cells ability to interfere in the epitelium selective permeability; the epitelium cells manifest the capacity to effect the absorption, secretion and excretion; such functions interfere on the epitelium permeability;
4) performance of the metabolic functions due to theis ability to effect hidrosalinic exchanges and to effect metabolytes transfers due to their cells and intracellular spaces high degree of poorly selective permeability;
5) transport of products along the epitelial surface due to the participation of the cilium;
6) sensorial perception and
7) germinative function.

Among these functions, the first four derive mostly from the epitelium cells selective permeability, over which are additionaly superimposed the additional affects corresponding to their properties of absorption, excretion and secretion. Among the general functions performed by the coating epiteliums, the selective permeability is responsible by the efficiency regarding the ability to coat, protect and isolate the surfaces, as well as to effect the control of the homeostasis; the passive absorption and the metabolytes transfer capacity are executed normally by the majority of the cells of these epiteliums, which demand only minor adaptations to become able to effectively perform such functions. On the contrary, the functions of absorption, excretion and secretion depend on properties that develop successively and would become paramount, mostly in some specialized types of coating epitelium, which adapted following a new and specific direction. The sensorial perception and the germinative function are more specific functions that are only manifest by certain epiteliums even more specialized. Considering their cell's morphological characteristics, the coating epiteliums have been classified according to the same number of cellular extracts they bear in: simple (a single extract) and stratified (two or more extracts). Both the simple epiteliums and the stratified ones, conforming to their cell's format, are in turn subdivided into pavementous, cubic or prismatic. The simple epiteliums are usually adapted to manifest wholy their most expressive fundamental property, that consists in their permeability, which degree and selectivity vary. The simple coating epiteliums, constituted by a single layer of pavementous or cubic-prismatic cells, present major differences regarding their functional properties, correlated not only to their cell's morphology, but also to the intracellular space's properties. The simple pavementous epiteliums are usually very pervious; the cubic-prismatic ones are less pervious. The coating epiteliums permeability, in addition to being selective, is controlled by their cell's functional activity, although the control looses efficiency in the same order as the intracellular space's permeability increases. The cubic-prismatic epiteliums, being less pervious than the pavementous, are more effective to control their permeability. Based on the format of the epitelium cell, in its permeability and the coating epiteliums most common adaptations, it is possible to generate a provisional classification for these epiteliums. Thus, the simple coating epiteliums are divided into two classes: pavementous and cubic-prismatic. Each class is subdivided according to it's functional properties in open or pervious epiteliums, in semi-occlusive or poorly-pervious and occlusive or impervious. In the simple coating epiteliums classification, the cubic epiteliums and the prismatic epiteliums are usually considered distinct, being defined and identified according to the format of the epitelium cells that make them up. However some functional studies have showed that the correlation between form and function presents several exceptions. For this reason a functional classification is adopted considering predominantly it's permeability. According to this criterium these epiteliums are denominated cubic-prismatic comprising the semi-oclusive and oclusive epiteliums. Following the same criterium the stratified stratified epiteliums can be subdivided into: pavementous and cubic-prismatic- The stratified epiteliums are adapted to perform primarily the mechanical protection function, because they are impervious or poorly pervious. The epiteliums comprise, in addition to the cells, the intercelular space and the base membrane, which interfere in their permeability degree; their permeability derives not only from their cell's peculiar properties, responsible for the transcelular permeability way, but also from the presence of another permeability way of their walls, constituting the intercelular or paracelular way. The transcelular way comprises two diferent ways that consist of the transmembranosa way and the transcanicular or trancitose way. It has been demonstrated, experimentally, that the coating epiteliums can be transposed by water and by substances of various natures, both through their epitelium cells (transcelular way) and through the .. way situated between their cells (intercelular way). In the first instance the epitelium cell can effect the permeability control of the epitelium through its biological activity, making this process selective. As for the intercelullar way permeability, the epitelium cell, although not behaving in a totally passive form, does not interfere directly in the transport selectivity. The sole form of cell active participation, in this instance, comprises the determination, exceptionally, the enlargement of the corresponding intercelular space. By means of the action of the microfilaments that constitute its cito-skeleton, the epitelium cell, specially those of certain types of simple coating epiteliums pavementous of the open type, can change its format and retract segments of its citoplasma; thus being able to influence the size of the intercellular space and regulate it. It has been established that the transcellular permeability of the simple coating epiteliums is perfectly distinct from the intercellular permeability, because both are subordinated to very different mechanisms. The epitelium cell permeability, which is selective, is influenced by its biological activity; on the contrary, the intercelular permeability is totally passive, and thus is not selective. Several experimental results have confirmed that the transposition of solutions through the epiteliums is subject to multiple control mechanisms, among which is paramount the intrinsec functional activity of its cells. On the contrary, the intercelular space permeability is generally not controlled, because in this case the transposition of a molecule through the epitelium follows only the corresponding physical laws and is directly related to its diameter, its electrical cargo and, obviously, to the intercellular space size; these three variables constitute the main limiting factors that interfere on the intercelular permeability of the simple coating epiteliums. The transcelular permeability of the simple coating epiteliums can be exercised through two distinct and independent ways: the transmembrane way, which is the true transcellular way, and the transcanicular way, which happens through the vesicles and the cannules or tubes of the vesículo-canalicular system, found inside the citoplasma of many types of coating epitelium cells". Consequently, the coating epiteliums are pervious, which allows the controlled and selective passage of various products through its wall. It is demonstrated that the permeability degree affects strongly the coating epiteliums function.

Three types of coating epiteliums are thus considered:
1- of wide permeability;
2- Of reduced permeability;
3- Of nule permeability.

The purpose is to prove through the formulation that there is an intense metabolic exchange demonstrating that the epitelium actuates on the transfer of metabolytes. This penetration of substances is complete and gradual and trespasses these epitelium layers until it penetrates the small blood vessels, reaching the circulatory current.

There is a description of the molecules to estimate the coating epiteliums permeability. Ex.: Hemoglobine, Ferritine, Lipo-proteines and enzymes.

Is is also known the transcitose on the transposition of the epiteliums by the macro and micro molecules until the vascular eye depending of their association.

The object of the present invention is the use of papain, vitamin-E and hyaluronidase for manufacturing of a **"PHARMACEUTICAL COMPOSITION COMPRISING CARRIERS FOR PRODUCTS BASED ON VITAMIN-E, PAPAIN AND HYALURONIDASE"**

The formulation of such pharmaceutical composition comprises particularly:

| | |
|---|---|
| PAPAIN - | 0.2 to 15% |
| HYALURONIDASE | 50 to 900 tru/mg |
| VITAMIN-E | 10 to 2000 mg |

More advantageously the formulation of the pharmaceutical composition comprises:

| | |
|---|---|
| PAPAIN - | 0.5 to 5% |
| HYALVRONIDASE | 50 to 900 tru/mg |
| VITAMIN-E | 10 to 2000 mg |

This technique was proofed through studies performed with 08 outpatients, in 02 distinct sessions of double blind analysis. The delimited area measured 15x10 cm with the application of gel, after 15 minutes the measurements were started through liquid chromatography coupled to mass spectometry.

The comparison of a confirm cream with the same active substance, for the purpose of calibration, yielded the following results regarding the cream according to the present invention, with an analytical type of equal absorption area:
Confirm cream = area 131 vol. 0,54 = 8 hours;
Tested cream (invention) = area 131 vol. 0,54 = 2 hours.

The penetration relation equals to 100% of the required cream.

### SOME SUBSTANCES TO BE CARRIED (MERELY FOR EXAMPLE) :

- CUTANEOUS CICATRIZATORS
- PURE ANTIBIOTICS AND SULFA DERIVATIVES
- TOPICAL DERMATOLOGIC ANTI-FUNGUS AGENT
- TOPICAL RUBIFACIENT ANTIREUMATICS GORTICOSTEROIDS, ANTIMICOTICS, PURE AND ASSOCIATED ANTIBACTERICIDES
- TOPICAL ANTI-VARIX
- ANTI-HISTAMINIC ANTI-ITCH
- TOPICAL ANTIVIRALS
- TOPICAL LOCAL ANESTHETICS
- HORMONAL AND NON-HORMONAL ANTI-INFLAMMATORY
- HISTAMINE CLOROHYDRATE
- FILDENAFIL CITRATE
- FENTOLAMINA MESILATE
- ALPROSTADIL (Prostaglandine)

## Claims

1. Use of Papain, vitamin E, and Hyaluronidase for manufacturing of a pharmaceutical composition containing a pharmacologically active product to be delivered through the skin wherein such composition contains as carriers of the product:
- Papain 0.2 to 15%
- Vitamin-E 10 to 2000 mg
- Hyaluronidase 50 to 900 tru/mg

2. Use of Papain, vitamin E and Hyaluronidase as claimed in Claim 1, wherein such composition advantageously contains as carriers of the product:
- Papain 0.5 to 15%
- Vitamin-E 10 to 2000 mg
- Hyaluronidase 50 to 900 tru/mg

## Patentansprüche

1. Verwendung von Papain, Vitamin E und Hyaluronidase zur Herstellung einer pharmazeutischen Rezeptur, umfassend eine pharmakologisch aktive Substanz, die über die Haut zugeführt wird, wobei die Rezeptur als Träger für die Substanz folgendes umfasst:
- Papain 0,2 bis 15 %
- Vitamin E 10 bis 2000 mg
- Hyaluronidase 50 bis 900 TRU/mg

2. Verwendung von Papain, Vitamin E und Hyaluronidase nach Anspruch 1, wobei die Rezeptur bevorzugt als Träger für die Substanz folgendes umfasst:
- Papain 0,5 bis 15 %
- Vitamin E 10 bis 2000 mg
- Hyaluronidase 50 bis 900 TRU/mg

## Revendications

1. Utilisation de papaïne, vitamine E et hyaluronidase pour la préparation d'une composition pharmaceutique comprenant un produit pharmacologiquement actif destiné à être absorbé à travers la peau, **caractérisée en ce que** ladite composition comprend en tant que vecteurs dudit produit :
- papaïne 0,2 à 15%
- vitamine E 10 à 2000 mg
- hyaluronidase 50 à 900 tru/mg

2. Utilisation de papaïne, vitamine E et hyaluronidase selon la revendication 1, **caractérisée en ce que** ladite composition comprend avantageusement en tant que vecteurs dudit produit :
- papaïne 0,5 à 15%
- vitamine E 10 à 2000 mg
- hyaluronidase 50 à 900 tru/mg
